# EUROPÄISCHE PATENTANMELDUNG

(11) **EP 2 703 021 A1**
(43) Veröffentlichungstag der Anmeldung: **05.03.2014**
(21) Anmeldenummer: 12182630.9
(22) Anmeldetag: 31.08.2012
(51) Int. Cl.: A61M 5/30, A61M 5/315

(54) **Zylinerkolbeneinheit mit zweiteiligem Kolben**

(71) Anmelder: LTS LOHMANN Therapie-Systeme AG, 56626 Andernach (DE)
(72) Erfinder: Dr. Spilgies, Heiko, 81373 München (DE); Hadaschik, Roman, 99817 Eisenach (DE); Dr. Wortmann, Uwe, 35037 Marburg (DE)

(57) **Zusammenfassung**

Die Erfindung betrifft eine Zylinder-Kolben-Einheit (100) eines Einweginjektors, die einen Zylinder (101) und einen darin verschiebbaren, einen Verdrängungsraum begrenzenden Kolben (111) umfasst. Der Kolben umfasst ein in Richtung des Verdrängungsraums zeigendes Abdichtkolbenelement (112) und ein dem Verdrängungsraum abgewandtes Führungskolbenelement (121). Der Elastizitätsmodul des Führungskolbenelements ist größer als der Elastizitätsmodul des Abdichtkolbenelements. Außerdem sind das Führungskolbenelement und das Abdichtkolbenelement mittels eines Schubgelenks miteinander verbunden.

Mit der vorliegenden Erfindung wird selbst nach langer Lagerung einer befüllten Zylinder-Kolben-Einheit die Sterilität einer Medikamentenlösung zu gewährleistet und das gleichförmige Ausbringen dieser Medikamentenlösung sichergestellt.

## Beschreibung

Die Erfindung betrifft eine Zylinder-Kolben-Einheit eines Einweginjektors, die einen Zylinder und einen darin verschiebbaren, einen Verdrängungsraum begrenzenden Kolben umfasst sowie einen Einweginjektor mit einer derartigen Zylinder-Kolben-Einheit.

Aus der DE 10 2009 031 303 A1 ist eine derartige Zylinder-Kolben-Einheit bekannt. Nach langer Lagerung einer befüllten Zylinder-Kolben-Einheit kann es zu einer ungleichförmigen Medikamentenausbringung kommen.

Der vorliegenden Erfindung liegt die Problemstellung zugrunde, auch nach langer Lagerung einer befüllten Zylinder-Kolben-Einheit die Sterilität einer Medikamentenlösung zu gewährleisten und das gleichförmige Ausbringen dieser Medikamentenlösung sicherzustellen.

Diese Problemstellung wird mit den Merkmalen des Hauptanspruchs gelöst. Dazu umfasst der Kolben ein in Richtung des Verdrängungsraums zeigendes Abdichtkolbenelement und ein dem Verdrängungsraum abgewandtes Führungskolbenelement. Der Elastizitätsmodul des Führungskolbenelements ist größer als der Elastizitätsmodul des Abdichtkolbenelements. Außerdem sind das Führungskolbenelement und das Abdichtkolbenelement mittels eines Schubgelenks miteinander verbunden.

Weitere Einzelheiten der Erfindung ergeben sich aus den Unteransprüchen und den nachfolgenden Beschreibungen schematisch dargestellter Ausführungsbeispiele.
- Figur 1:: Einweginjektor vor dem Auslösen und beim Auslösen;
- Figur 2:: Detail aus Figur 1;
- Figur 3:: Einweginjektor nach dem Auslösen;
- Figur 4:: Detail aus Figur 3;
- Figur 5:: Einweginjektor bei entleerter Zylinder-Kolbeneinheit;

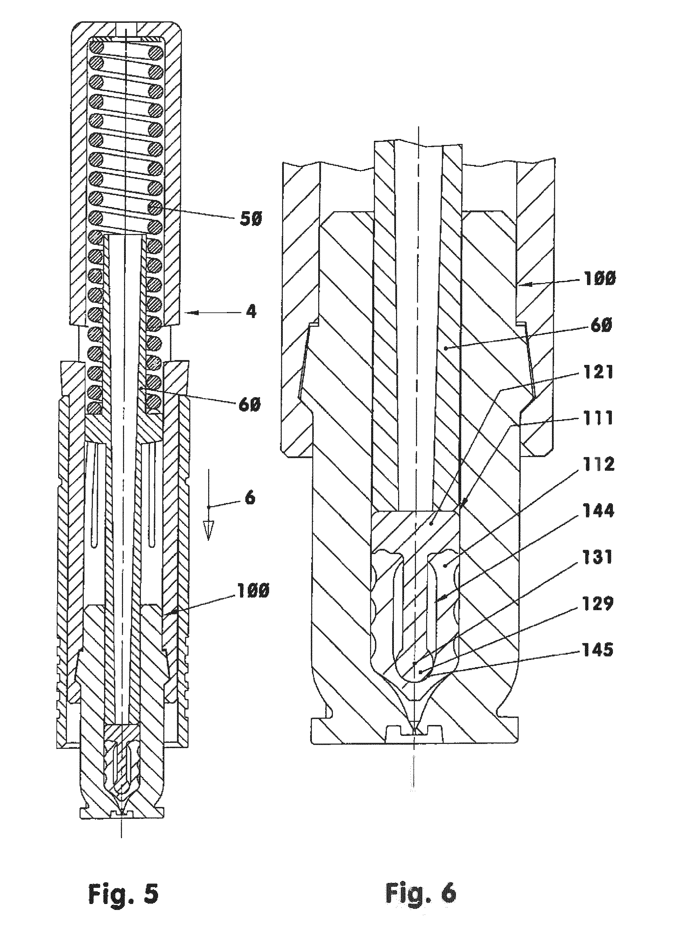

- Figur 5:: Detail aus Figur 5;

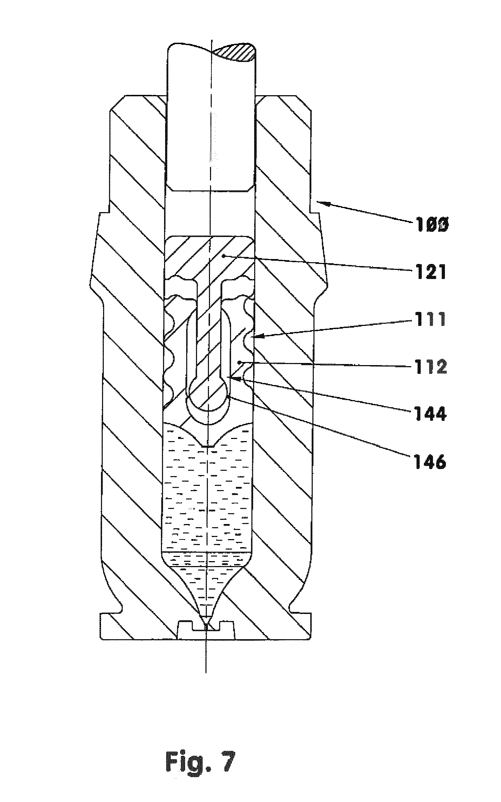

- Figur 7:: Zylinder-Kolbeneinheit mit vorgeformtem Abdichtkolbenelement.

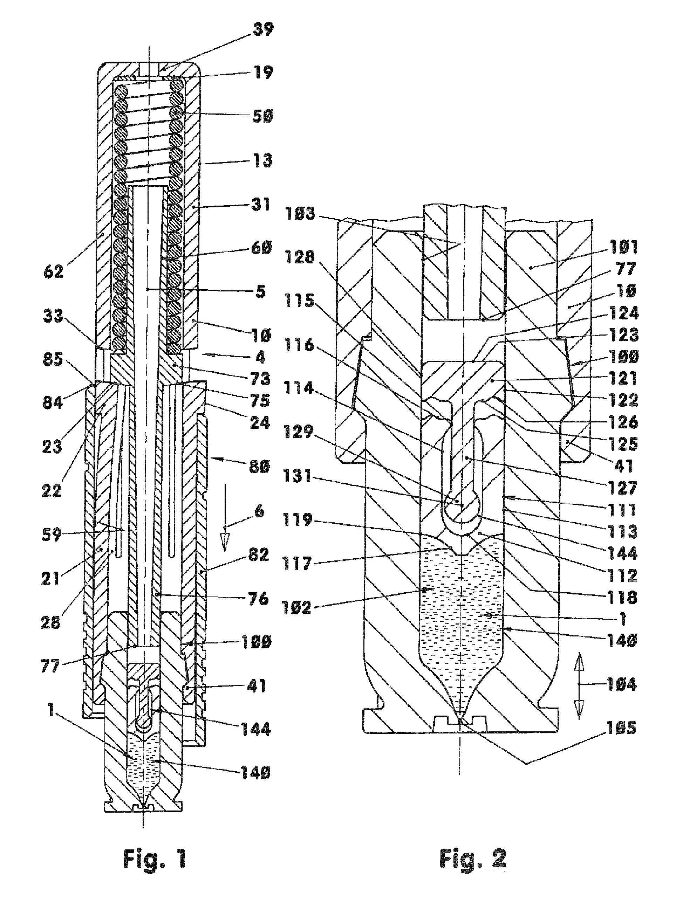

Die Figur 1 zeigt einen Einweginjektor (4) mit einem Gehäuse (10), einer Auslöseinheit (80) und einer Zylinder-Kolbeneinheit (100). Letztere sitzt beispielsweise im unteren Bereich des Gehäuses (10) und ragt aus diesem heraus. In dem in der Figur 1 dargestellten Längsschnitt eines Injektors (4) zeigt die linke Seite der Schnittdarstellung den Injektor (4) vor der Betätigung der Auslöseeinheit (80). Auf der rechten Seite ist das Auslöseelement (82) in der Auslösebewegungsrichtung (6), also nach unten, verschoben.

Das einteilige Gehäuse (10) ist topfförmig ausgebildet und hat eine z.B. zylinderförmige Außenfläche (13). Es verfügt im Ausführungsbeispiel über zwei einander gegenüber angeordnete Durchbrüche (33) und über Druckstäbe (21). Die oberen Enden der Druckstäbe (21), die Nocken (22), stützen sich in dem hier links dargestellten Zustand des Einweginjektors (4) vor dem Beginn des Auslösens an der Innenseite eines Auslöseelements (82) der Auslöseeinheit (80) ab. Ein federbelasteter Kolbenbetätigungsstempel (60) stützt sich an den Druckstäben (21) des Gehäuses (10) ab.

Das Auslöseelement (82) ist hülsenartig ausgebildet und umgibt längsverschieblich den unteren Bereich der z.B. zylindrischen Außenfläche (13) des Gehäuses (10). Im Ausführungsbeispiel hat das Auslöseelement (82) eine kreisringförmige Querschnittsfläche. Die Querschnittsflächen des Gehäuses (10) und des Auslöseelements (82) können auch durch ein Rechteck, Sechseck, eine Ellipse, etc. begrenzt sein.

Das Gehäuse (10) ist ein unten offener Hohlkörper mit obenliegendem Boden (39). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das ist zum einen der obere Mantelbereich (31) und zum anderen der untere Fixierbereich (41). Im Gehäuse (10) sind ein Kolbenbetätigungsstempel (60) und eine Schraubendruckfeder (50) als Federenergiespeicher angeordnet.

Am unteren Rand des einzelnen Durchbruchs (33) ist jeweils ein Druckstab (21) als elastischer Biegebalken angeformt. Die Anformstelle für die Druckstäbe (21) liegt knapp oberhalb des Fixierbereichs (41). Zur Ausbildung des jeweiligen Druckstabs (21) befindet sich im unteren Bereich des Mantelabschnitts (31) ein schmaler, zumindest annähernd u-förmiger Spalt, der den einzelnen Druckstab (21) seitlich und oben umgibt.

Der Druckstab (21) hat beispielsweise auf 80 % seiner Länge die Wandstärke und die Krümmung der Wandung des Gehäuses (10). Dieser Bereich hat unter anderem auch die Funktion eines federelastischen Biegebalkens (28). Er hat einen sichelförmigen Querschnitt. Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zweier oder mehrerer Druckstäbe (21) auch nur ein einziger Druckstab (21) verwendet werden.

Das hier obere Ende des einzelnen Druckstabs (21) wird durch den radial nach außen abstehenden Nocken (22) gebildet. Letzterer hat zumindest eine in Richtung der Mittellinie (5) orientierte Abstützfläche (23) und eine der Mittellinie (5) abgewandte Anlagefläche (24).

Im unteren Bereich des Gehäuses (10) befinden sich Halteelemente zur Befestigung der Zylinder-Kolben-Einheit (100). Die Zylinder-Kolben-Einheit (100) besteht im Ausführungsbeispiel aus einem, mit einer Injektionslösung befüllbaren, transparenten Zylinder (101) und einem darin gelagerten Kolben (111). In der Darstellung der Figur 1 sitzt der Kolben (111) in der hinteren Position. Der Kolben (111) begrenzt einen mit einer Medikamentenlösung (1) befüllbaren Verdrängungsraum (140). Oberhalb des Kolbens (111) ist im Gehäuse (10) der Kolbenbetätigungsstempel (60) z.B. so angeordnet, dass er den Kolben (111) zwar nicht berührt, jedoch mit seinem unteren Ende z.B. im oberen Bereich des Zylinders (101) seitlich geführt wird.

Die Figur 2 zeigt die Zylinder-Kolbeneinheit (100) aus der Figur 1. Der Zylinder (101) hat im Ausführungsbeispiel einen weitgehend zylinderförmigen Zylinderinnenraum (102), der sich im unteren Bereich zur verschließbaren Austrittsöffnung (105) hin verjüngt. Der Zylinder (101) kann auch eine quadratische, sechseckige, etc. Innen- und/oder Außenquerschnittsfläche aufweisen.

Der im Zylinderinnenraum (102) angeordnete Kolben (111) ist zweiteilig aufgebaut. Er umfasst ein in den Darstellungen der Figuren 1 und 2 unten angeordnetes Abdichtkolbenelement (112) und ein in der Auslösebewegungsrichtung (6) hinter diesem angeordnetes Führungskolbenelement (121). Das Abdichtkolbenelement (112) ist damit dem Verdrängungsraum (140) zugewandt und das Führungskolbenelement (121) ist dem Verdrängungsraum (140) abgewandt angeordnet.

Das Führungskolbenelement (121) ist beispielsweise aus einem schlagzähen oder geringelastischen Werkstoff, beispielsweise einem Kunststoff, hergestellt. Dies kann z.B. Polycarbonat (PC) Acrylnitrid-Butadien-Styrol-Copolymer (ABS), etc. sein. Es ist auch denkbar, das Führungskolbenelement (121) aus Stahl, Aluminium oder aus Legierungen dieser Werkstoffe herzustellen. Der Elastizitätsmodul des Werkstoffs des Führungskolbenelements (121) liegt zwischen zwei Gigapascal und 220 Gigapascal. Die beispielsweise zylinderförmige Mantelfläche (122) hat im Ausführungsbeispiel einen Durchmesser, der bis um zwei zehntel Millimeter kleiner ist als der Innendurchmesser des Zylinders (101). Die Zylinderinnenwandung (103) und die Mantelfläche (122) bilden damit eine Spielpassung. Die Länge der Mantelfläche (122) in der Zylinderlängsrichtung (104) beträgt hier ein Drittel des Duchmessers des Führungskolbenelements (121). Diese Länge kann jedoch auch größer als der doppelte Durchmesser des Führungskolbenelements (121) sein. Die Zylinderlängsrichtung (104) ist bei eingebauter Zylinder-Kolbeneinheit (100) in der Längsrichtung (5) des Einweginjektors (4) orientiert.

Die dem Kolbenbetätigungsstempel (60) zugewandte Rückseite (123) des Führungskolbenelements (121) ist z.B. bereichsweise als Planfläche ausgebildet. Im Ausführungsbeispiel ist sie im Randbereich abgerundet. Sie bildet eine Anschlagfläche (124) für den Kolbenbetätigungsstempel (60). Die dem Abdichtkolbenelement (112) zugewandte Vorderseite (125) des Führungskolbenelements (121) hat eine ringförmige Ausnehmung (126) sowie einen zentralen Zapfen (127). Die Querschnittsfläche dieses Zapfens (127), dessen Länge beispielsweise dem Dreifachen der Länge des zylinderförmigen Abschnitts (128) entspricht, beträgt im Ausführungsbeispiel 7 % der normal zur Zylinderlängsrichtung (104) orientierten Querschnittsfläche des Führungskolbenelements (121). Das freie Ende des Zapfens (127) ist kugelförmig ausgebildet. Der Durchmesser der Kugel (129) beträgt beispielsweise zwischen 35 % und 50 % des Durchmessers des Führungskolbenelements (121).

Das Abdichtkolbenelement (112) ist aus einem Elastomerwekstoff hergestellt. Dieser elastisch verformbare Kunststoff kann z.B. ein Pharmagummi sein. Sein Elastizitätsmodul ist geringer als der Elastizitätsmodul des Führungskolbens (121). Beispielsweise ist der Elastizitätsmodul dieses Werkstoffs kleiner oder gleich zwei Gigapascal. Die Umfangsfläche (113) des Abdichtkolbenelements (112) bildet mit der Innenwandung (103) des Zylinders (101) zumindest bereichsweise eine Presspassung. Die normal zur Längsrichtung (104) orientierte Querschnittsfläche des Abdichtkolbenelements (112) ist damit größer als die normal zur Längsrichtung (104) orientierte Querschnittsfläche des Führungskolbenelements (121). Das Abdichtkolbenelement (112) umfasst eine in der Längsrichtung (104) orientierte langlochartige Ausnehmung (114). Diese grenzt mit einem Einführring (115) an die zum Führungskolbenelement (121) gerichtete Rückseite (116) des Abdichtkolbenelements (112). Die Länge der langlochartigen Ausnehmung (114) entspricht beispielsweise zwei Drittel der Länge des Abdichtkolbenelements (112).

In den Darstellungen der Figuren 1 und 2 ist die Rückseite (116) des Abdichtkolbenelements (112) beabstandet von der Vorderseite (125) des Führungskolbenelements (121). Die Kolbenspitze (117) ist im Ausführungsbeispiel kegelförmig ausgebildet, wobei der Spitzenwinkel ein stumpfer Winkel ist.

Die Kugel (129) des Führungskolbenelements (121) bildet ein Vollprisma (129) und die langlochartige Ausnehmung (114) des Abdichtkolbenelements (112) ist ein Hohlprisma (114). Das Hohlprisma (114) und das Vollprisma (129) bilden als Elementenpaar ein Schubgelenk (144). Dieses hat einen in der Längsrichtung (104) der Zylinder-Kolbeneinheit (100) orientierten Freiheitsgrad. Der Resthub des Vollprismas (129) aus den Figuren 1 und 2 zur vorderen Anlagefläche (118) des Hohlprismas (114) ist z.B. kleiner oder gleich dem Abstand der Rückseite (116) des Abdichtkolbenelements (112) zur Vorderseite (125) des Führungskolbenelements (121).

Gegebenenfalls kann der Zapfen (127) reibschlüssig in der Einführöffnung (115) sitzen. Selbst bei senkrecht aufgestelltem Einweginjektor (4) gleitet das Führungskolbenelement (121) nicht selbstständig in Richtung des Abdichtkolbenelements (112). Es ist auch denkbar, in der langlochartigen Ausnehmung (114) einen Rastring anzuordnen, der bei unbelastetem Führungskolbenelement (121) dieses in der vom Abdichtkolbenelement (112) beabstandeten Grundposition hält. Ein derartiger Rastring (146) ist beispielsweise in der Figur 7 dargestellt.

Das Auslöseelement (82) ist z.B. aus Acrylnitril-Butadien-Styrol-Copolymer (ABS) hergestellt. Es endet rückwärtig mit einer scharfen Kante (85), die Teil einer stirnseitigen Rücksprungflanke (84) des Auslöseelements (82) ist. Unterhalb der Kante (85) berühren in der linken Darstellung der Figur 1 die Druckstäbe (21) angeformte Nocken (22) mit ihren außen liegenden Anlageflächen (24) sichernd die Innenwandung (59) des Auslöseelements (82).

Der im Gehäuse (10) angeordnete Kolbenbetätigungsstempel (60) ist in zwei Bereiche aufgeteilt. Der untere Bereich ist der Kolbenschieber (76). Sein Durchmesser ist etwas kleiner als der Innendurchmesser des hinteren Bereichs des Zylinders (101). Die untere Stirnfläche des Kolbenschiebers (76) wirkt direkt auf den Kolben (111).

Der obere Bereich des Kolbenbetätigungsstempels (60) , der Stempelteller (73), ist eine flache, zumindest bereichsweise zylindrische Scheibe, deren Außendurchmesser einige zehntel Millimeter kleiner ist als der Innendurchmesser des Gehäuses (10) im Mantelbereich (31). Die untere Stirnseite weist eine um den Kolbenschieber (76) herum angeordnete Bundfläche (75) auf. Sie hat beispielsweise die Form eines Kegelstumpfmantels, dessen Spitzenwinkel ca. 100 bis 140 Grad beträgt. Im dargestellten Ausführungsbeispiel hat die Bundfläche (75) einen Spitzenwinkel von 140 Grad. Die gedachte Spitze des Kegelstumpfmantels liegt auf der Mittellinie (5) im Bereich des Kolbenschiebers (76). Die Bundfläche (75) kann sphärisch gekrümmt sein, sie kann einzelne, schräg zueinander angeordnete Ebenen aufweisen, etc.

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Schraubendruckfeder (50) stützt sich am oben liegenden Boden (39) des Gehäuses (10) unter Zwischenschaltung einer Distanzhülse (19) ab. Die Federkraft der Schraubendruckfeder (50) wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

Der Kolbenbetätigungsstempel (60) hat oberhalb des Stempeltellers (73) einen Führungszapfen (62). Letzterer führt die Schraubendruckfeder (50) oder wird durch diese geführt. Unterhalb des Stempeltellers (73) befindet sich zentral in der Verlängerung des Führungszapfens (62) der Kolbenschieber (76), der bei einer Betätigung des Einmal-Injektors (4) auf den Kolben (111) wirkt. Der Kolbenschieber (76) hat eine Stirnfläche (77), mit der er beim Auslösen die komplementär geformte Stirnfläche des Kolbens (111) kontaktiert.

Bevor der Einweginjektor gefüllt und eingesetzt wird, ist der Federenergiespeicher (50) vorgespannt, vgl. Figur 1, linker Teilschnitt. Die beiden auf Druck belasteten Druckstäbe (21) halten den Kolbenbetätigungsstempel (60) an dessen Stempelteller (73) in seiner vorgespannten Lage.

Die Biegeelastizität der Druckstäbe (21) und der Stempelteller (73) drücken die Stützstäbe (21) zumindest annähernd radial nach außen gegen das Auslöseelement (82). Dort liegen sie über Nocken (22) am Auslöseelement (82) an.

Um den Einweginjektor benutzen zu können, muss zunächst die zylinder-Kolben-Einheit (100) befüllt werden. Dazu wird z.B. ein Stopfen aus der Adapteröffnung (105) entfernt und eine Injektionslösung z.B. eingepresst oder eingesaugt. Der Kolben (111) wird hierbei zurückgedrückt oder zurückgezogen.

Der Einweginjektor (4) kann nun beispielsweise während eines zeitintervalls von drei bis fünf Jahren gelagert werden. Das Abdichtkolbenelement (112) sichert die Sterilität der Medikamentenlösung (1).

Zum Auslösen des Einweginjektors (4) kann das Auslöseelement (82) in Richtung der Zylinder-Kolben-Einheit (100) verschoben werden, vgl. Figur 1, rechter Teilschnitt. Der in diesem Teilschnitt dargestellte Zustand ist nicht statisch, er wird daher im Folgenden als fiktiver Zustand bezeichnet.

Beim Auslösen des Einweginjektors (4) gleitet das Auslöseelement (82) auf der Außenwandung (13) des Gehäuses (10) in der Auslösebewegungsrichtung (6) linear nach unten, also in Richtung der Injektionsstelle. Der Kolbenbetätigungsstempel (60) verdrängt, angetrieben vom Federelement (50), den Druckstab (21).

Die Anlageflächen (24) der Druckstäbe (21) rutschen über die Kante (85). Die Druckstäbe (21) biegen sich elastisch nach außen in ihre eigentliche Ausgangslage und springen unter der Kraft des Federelements (50) radial nach außen.

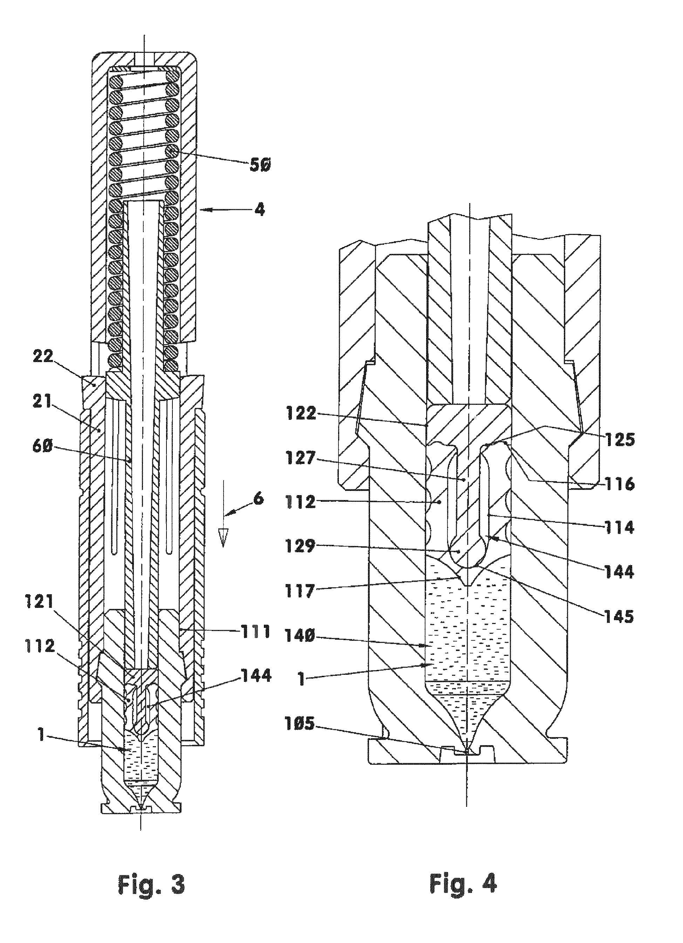

Sobald die Nocken (22) in die Aufweitung verdrängt sind, schnellt der Kolbenbetätigungsstempel (60) ungehindert nach unten. Der Kolbenbetätigungsstempel (60) trifft auf die Anschlagfläche (124) des Führungskolbenelements (121), vgl. die Figuren 3 und 4. Das Führungskolbenelement (121) wird in Richtung der Austrittsöffnung (105) des Zylinders (101) beschleunigt. Hierbei gleitet das Führungskolbenelement (121) praktisch reibungsfrei oder mit nur geringer Reibung entlang der Zylinderinnenwandung (103).

Das nach vorne schnellende Führungskolbenelement (121) trifft mit der Kugel (129) auf die vorne liegende Anlagefläche (118) der langlochförmigen Ausnehmung (114) des Abdichtkolbenelements (112) auf. In der vorderen Hubendlage (145) ist das Vollprisma (129) näher an der Kolbenspitze (117) - und damit näher an der Ausstoßöffnung (105) - als der Massenschwerpunkt des Abdichtkolbenelements (112). Das Massenträgheitsmoment der beschleunigten Masse des Kolbenbetätigungsstempels (60) und des steifen Führungskolbenelements (121) trifft auf das zunächst ruhende Abdichtkolbenelement (112). Das Abdichtkolbenelement (112) wird elastisch verformt und beschleunigt. Hierbei wird es beispielsweise in Längsrichtung (104) gedehnt, während die Mantelfläche (113) zunächst noch beispielsweise vollflächig an der Zylinderinnenwandung (103) haftet. Aufgrund der Krafteinwirkung des Führungskolbenelements (121) wird die Mantelfläche (113) in Teilbereichen von der Zylinderinennwandung (103) entlastet und kann auch von dieser bereichsweise gelöst werden. Hierbei bewirkt u.a. der im Zylinderinnenraum (102) entstehende Flüssigkeitsdruck nach dem Prinzip der Selbsthilfe ein Anpressen des an die Kolbenspitze (117) angrenzenden Bereichs der Mantelfläche (122) des Abdichtkolbenelements (112) an die Zylinderinnenwandung (103). Beispielsweise nimmt - unter Beibehaltung der Dichtwirkung - die Flächenpressung des Abdichtkolbenelements (112) auf die Zylinderinnenwandung (103) ab. Das Abdichtkolbenelement (112) wird im Verdrängungsraum (140) in Richtung des Austrittsquerschnitts (105) verschoben und verdrängt die Flüssigkeit (1) durch den Austrittsquerschnitt (105) beispielsweise in die Haut des Patienten. Der Kolben (111) gleitet nun, angetrieben von der sich entspannenden Feder (50) mittels des Kolbenbetätigungsstempels (60), weitgehend ruckfrei und mit geringem Widerstand in die vordere Endlage. Hierbei ist der Verdrängungsraum (140) wirksam abgedichtet. Der Zylinder (100) wird entleert.

Der Impuls des Kolbenbetätigungsstempels (60) wird damit weitgehend verlustfrei auf den Inhalt des Verdrängungsraums (140) übertragen. Somit gelingt es mittels des Austrittsstrahls, die Haut des Patienten wirksam zu penetrieren. Selbst nach einer langen Lagerung der befüllten Zylinder-Kolben-Einheit (100) ist damit ein wirksames und sicheres Auslösen und Entleeren der Medikamentenkammer gegeben.

Die Figuren 5 und 6 zeigen den Kolben (211) in der unteren Endlage des Zylinders (101). Die Schraubenfeder (50) ist entspannt und der Kolbenbetätigungsstempel (60) drückt weiterhin gegen die Rückseite (123) des Führungskolbenelements (121). Die Vorderseite (125) des Führungskolbenelements (121) liegt an der Rückseite (116) des Abdichtkolbenelements (112) an und drückt dieses zusätzlich in Richtung der Austrittsöffnung (105). Die Mantelfläche (113) des Abdichtkolbenelements (112) liegt jetzt mit ringförmigen Abschnitten an der Zylinderinnenwandung (103) an. Die dazwischen liegenden Abschnitte der Mantelfläche (113) haben sich von der Zylinderinnenwandung (103) gelöst. Der Zylinder (101) ist entleert.

Das Schubgelenk (144) kann derart ausgebildet sein, dass der gedachte Kegel, dessen Mantellinien die Kolbenlippe (119) und dem Mittelpunkt (131) des Vollprismas (129) verbinden, einen Spitzenwinkel aufweist, der kleiner oder gleich 90 Grad ist. Die Mittellinie dieses Kegels ist hierbei in Längsrichtung des Kolbens (111) orientiert. Beim Verfahren des Abdichtkolbens (112) wird damit die Kolbenlippe (119) belastet.

Es ist auch denkbar, dass an der Anlagefläche (118) der Ausnehmung (114) eine beispielsweise metallische Verstärkung eingelegt ist. Diese kann in den Werkstoff des Abdichtkolbenelements (112) z.B. einvulkanisiert sein.

Die Figur 7 zeigt eine weitere Bauform einer Zylinder-Kolbeneinheit (100) mit einem zweiteiligen Kolben (111). Hierbei hat das unverformte Abdichtkolbenelement (112) eine schraubengangförmige Mantelfläche (113). In der Längsrichtung (104) hat die Mantelfläche (113) des Abdichtkolbenelements (112) damit eine wellenförmige Kontur. Beim Auftreffen des Kolbenbetätigungsstempels (60) wird der Kolben (111) nach vorne geschoben. Das Führungskolbenelement (121) trifft auf das Abdichtkolbenelement (112) und verschiebt dieses in Richtung der Austrittsöffnung (105). Das Abdichtkolbenelennent (112) wird z.B. derart verformt, dass die Flächenpressung des Abdichtkolbenelements (112) an der Zylinderinnenwandung (103) - unter Beibehaltung der Abdichtung - abnimmt.

Der in den Figuren 1, 3 und 5 dargestellte Einweginjektor (4) ist beispielsweise nadellos ausgebildet. Ebenso zeigen die Figuren 2, 4, 6 und 7 Zylinder-Kolbeneinheiten (100) für nadellose Einmalinjektoren (4). Die Injektoren können aber auch mindestens eine Nadel oder mindestens eine Kanüle umfassen.

Selbstverständlich ist es auch denkbar, die verschiedenen genannten Ausführungsformen miteinander zu kombinieren.

### Bezugszeichenliste:

- 1: Medikamentenlösung
- 4: Einweginjektor
- 5: Mittellinie des Injektors, Längsrichtung
- 6: Auslösebewegungsrichtung von (82), Abwärtsbewegung Richtungspfeil

- 10: Gehäuse, einteilig
- 13: Außenfläche, zylindrisch
- 19: Distanzhülse

- 21: Druckstäbe, Stützstäbe; Zughaken
- 22: Nocken
- 23: Abstützfläche
- 24: Anlagefläche
- 28: Biegebalken

- 31: Mantelbereich
- 33: Durchbrüche
- 39: Boden
- 41: Fixierbereich für die Zylinder-Kolben-Einheit

- 50: Federelement, Schraubendruckfeder, Federenergiespeicher
- 59: Innenwandung von (82)

- 60: Kolbenbetätigungsstempel
- 62: Führungszapfen

- 73: Stempelteller
- 75: Bundfläche, konisch
- 76: Kolbenschieber
- 77: Kolbenschieber-Stirnfläche, kegelmantelförmig

- 80: Auslöseeinheit
- 82: Auslöseelement
- 84: Rücksprungflanke
- 85: Kante, scharfkantig

- 100: Zylinder-Kolben-Einheit
- 101: Zylinder
- 102: Zylinderinennraum
- 103: Zylinderinnenwandung
- 104: zylinderlängsrichtung
- 105: Adapteröffnung, Austrittsöffnung

- 111: Kolben
- 112: Abdichtkolbenelement
- 113: Umfangsfläche, Mantelfläche
- 114: Ausnehmung, Hohlprisma
- 115: Einführring, Einführöffnung
- 116: Rückseite
- 117: Kolbenspitze
- 118: Anlagefläche
- 119: Kolbenlippe

- 121: Führungskolbenelement
- 122: Mantelfläche
- 123: Rückseite
- 124: Anschlagfläche
- 125: Vorderseite
- 126: Ausnehmung, ringförmig
- 127: Zapfen
- 128: zylinderförmiger Abschnitt
- 129: Kugel, Vollprisma

- 131: Mittelpunkt von (129)

- 140: Verdrängungsraum

- 144: Schubgelenk
- 145: Hubendlage
- 146: Rastring

## Patentansprüche

1. Zylinder-Kolben-Einheit (100) eines Einweginjektors (4), die einen Zylinder (101) und einen darin verschiebbaren, einen Verdrängungsraum (140) begrenzenden Kolben (111) umfasst, **dadurch gekennzeichnet,**
- **dass** der Kolben (111) ein in Richtung des Verdrängungsraums (140) zeigendes Abdichtkolbenelement (112) und ein dem Verdrängungsraum (140) abgewandtes Führungskolbenelement (121) umfasst,
- **dass** der Elastizitätsmodul des Führungskolbenelements (121) größer ist als der Elastizitätsmodul des Abdichtkolbenelements (112) und
- **dass** das Führungskolbenelement (121) und das Abdichtkolbenelement (112) mittels eines Schubgelenks (144) miteinander verbunden sind.

2. Zylinder-Kolben-Einheit (100) nach Anspruch 1, **dadurch gekennzeichnet, dass** das Führungskolbenelement (121) auf seiner dem Abdichtkolbenelement (112) abgewandten Rückseite (123) eine Anschlagfläche (124) aufweist.

3. Zylinder-Kolben-Einheit (100) nach Anspruch 1, **dadurch gekennzeichnet, dass** die Vorderseite (125) des Führungskolbenelements (121) und die Rückseite (116) des Abdichtkolbenelements (112) voneinander beabstandet sind.

4. Zylinder-Kolben-Einheit (100) nach Anspruch 1, **dadurch gekennzeichnet, dass** der Freiheitsgrad des Schubgelenks (144) in Längsrichtung (104) des Zylinders (101) orientiert ist.

5. Zylinder-Kolben-Einheit (100) nach Anspruch 1, **dadurch gekennzeichnet, dass** der Hub des Schubgelenks (144) kleiner ist als der Abstand der Vorderseite (125) des Führungskolbenelements (121) zur Rückseite (116) des Abdichtkolbenelements (112).

6. Zylinder-Kolben-Einheit (100) nach Anspruch 1, **dadurch gekennzeichnet, dass** die normal zur Längsrichtung (104) orientierte Querschnittsfläche des Abdichtkolbenelements (112) größer ist als die normal zur Längsrichtung (104) orientierte Querschnittsfläche des Führungskolbenelements (121).

7. Zylinder-Kolben-Einheit (100) nach Anspruch 1, **dadurch gekennzeichnet, dass** die Mantelfläche (113) des Abdichtkolbenelements (112) eine in Längsrichtung (104) orientierte wellenförmige Kontur hat.

8. Zylinder-Kolben-Einheit (100) nach Anspruch 1, **dadurch gekennzeichnet, dass** das Schubgelenk (144) ein Vollprisma (129) und ein Hohlprisma (114) umfasst und dass bei betätigtem Kolben (111) das Vollprisma (129) näher an der Ausstoßöffnung (105) liegt als der Massenschwerpunkt des Abdichtkolbenelements (112).

9. Einweginjektor (4) mit einer Zylinder-Kolben-Einheit (100) nach Anspruch 1 **dadurch gekennzeichnet, dass** er eine Auslösevorrichtung (80) umfasst.

10. Einweginjektor (4) nach Anspruch 9, **dadurch gekennzeichnet, dass** mittels der Auslösevorrichtung (80) eine auf einen Kolbenbetätigungsstempel (60) wirkende Feder (50) freigebbar ist.
